# EUROPÄISCHE PATENTSCHRIFT

(11) **EP 0 306 442 B1**
(45) Veröffentlichungstag und Bekanntmachung des Hinweises auf die Patenterteilung: **02.03.1994**
(21) Anmeldenummer: 88730200.8
(22) Anmeldetag: 05.09.1988
(51) Int. Cl.: A61N 1/05

(54) **Implantierbare Elektrode zur Herzstimulation**
Implantation electrode for heart stimulation
Electrode implantable de stimulation cardiaque

(30) Priorität: 03.09.1987 DE 8712041 U
(43) Veröffentlichungstag der Anmeldung: 08.03.1989
(73) Patentinhaber: BIOTRONIK Mess- und Therapiegeräte GmbH & Co Ingenieurbüro Berlin, D-12359 Berlin (DE)
(72) Erfinder: Kormann, Décio, S., Paraiso Sao Paulo (BR)
(74) Vertreter: Christiansen, Henning, Dipl.-Ing.

(56) Entgegenhaltungen:
- CH-B- 656 313
- GB-A- 2 157 954
- US-A- 4 161 952
- US-A- 4 481 953
- US-A- 4 534 366

## Beschreibung

Die Erfindung betrifft eine implantierbare Elektrode der im Oberbegriff des Anspruchs 1 angegebenen Art.

Implantierbare Elektroden dienen dazu, die von einem Herzschrittmacher abgegebenen Stimulationsimpulse auf das Herz eines Patienten zu übertragen, und ermöglichen darüber hinaus die Wahrnehmung der intrakardialen elektrischen Potentiale. Die Elektrode besteht aus einem Stecker, einem Kabel und einem Elektrodenkopf, der die Verbindung zum Myokard oder zum Brustbein herstellt. Den Hauptanteil einer Elektrode nimmt der Elektrodenleiter ein, der aus einem metallischen Leiter besteht, der nach außen hin isoliert ist.

Zur Erhöhung der Bruchfestigkeit von Elektrodenleitern werden vorzugsweise mehrwendelige Elektrodenleiter verwendet, bei denen mehrere dünnere Drahtwendeln entweder koaxial oder nebeneinander gewickelt sind. Durch die Verwendung von mehreren Wendeln konnten der Krümmungsradius und der Durchmesser der Wendel vergrößert werden, da die Häufigkeit des Elektrodenbruches umso größer ist, je geringer der Krümmungsradius und je kleiner der Durchmesser der Drahtwendel sind. Ein weiterer Vorteil mehrwendeliger Elektrodenleiter besteht in der Schaffung eines Lumen zum Einführen eines Mandrin zum Plazieren des Elektrodenkopfes.

Am Ende des Elektrodenleiters ist ein elektrisch leitfähiger Bereich zur Übertragung der Stimulationsimpulse vorgesehen, der mit einem vorzugsweise halbkugelförmigen Elektrodenkopf versehen ist.

Form und Größe sowie Art des verwendeten Materials des leitfähigen Bereichs der Elektrode haben einen wesentlichen Einfluß auf die Reizschwellenkurve. So ist bei Elektroden mit sehr kleinen Oberflächen zwar die akute und chronische Reizschwelle günstiger als bei Elektroden mit größeren Oberflächen, jedoch ist die Gefahr von Mikrodislokationen deutlich erhöht. Darüberhinaus wurde festgestellt, daß gesinterte, poröse oder aktivierte Oberflächen im Gegensatz zu glatten Oberflächen das Einwachsen einer dünnen bindegewebigen Verankerung erlauben und sowohl im Hinblick auf ihr Sensingverhalten als auch bezüglich der Reizschwelle bessere Eigenschaften aufweisen als Elektroden, bei denen die stimulierende Oberfläche aus einem glatten Metall besteht. Damit die Elektrode eine möglichst geringe Dislokationsneigung zeigt, wurden verschiedene Fixationsmechanismen entwickelt, bspw. in Form eines Kragens, der der Verankerung der Elektrodenspitze dient oder in Form von aktiven Fixationsmechanismen wie Federn, Schrauben oder Fortsätzen aus dem Isolationsmaterial der Elektroden.

Aus US 4 161 952 ist eine implantierbare Elektrode zur Herzstimulation nach dem Oberbegriff des Anspruchs 1 bekannt.

Diese Elektrode ist zur Verbindung mit einem einfachen, nicht gewendelten Elektrodenleiter bestimmt und sehr aufwendig in der Herstellung.

Aufgabe der vorliegenden Erfindung ist es, eine implantierbare Elektrode zur Herzstimulation zu schaffen, die leicht positionierbar ist, optimale Reizschwelleneigenschaften bei geringer Dislokationsneigung aufweist, sowie einfach herstell- und mit einem gewendelten Elektrodenleiter verbindbar ist.

Diese Aufgabe wird erfindungsgemäß durch eine implantierbare Elektrode mit den Merkmalen des Patentanspruchs 1 gelöst.

Der wesentliche Vorteil der erfindungsgemäßen Elektrode ist, daß sie einerseits wegen der zylindrischen Form des leitfähigen Bereichs insbesondere im Bereich des Brustbeins eines Herzschrittmacher - Patienten leicht positionierbar ist und andererseits wegen der Anordnung eines wendel- oder spiralförmig gestreckten Leiters sowohl optimale Reizschwelleneigenschaften als auch geringe Dislokationsneigungen hat, da ähnlich wie bei gesinterten, porösen oder aktivierten Oberflächen das Einwachsen einer dünnen bindegewebigen Verankerung möglich und damit die Gefahr von Mikrodislokationen deutlich verringert wird. Ein weiterer Vorteil der erfindungsgemäßen Elektrode besteht in ihrer einfachen und preisgünstigen Herstellbarkeit und in einer leichten, haltbaren Verbindung mit dem Elektrodenleiter.

Vorteilhaft ist dabei insbesondere, daß mit einfachen Mitteln verschiedene Elektrodenvarianten mit unterschiedlicher Länge und unterschiedlichem Durchmesser des leitfähigen Bereichs hergestellt werden können.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine teilweise geschnittene Ansicht der erfindungsgemäßen Elektrode und
Figur 2 bis 7 die einzelnen Stufen zur Herstellung einer Elektrode gemäß Figur 1.

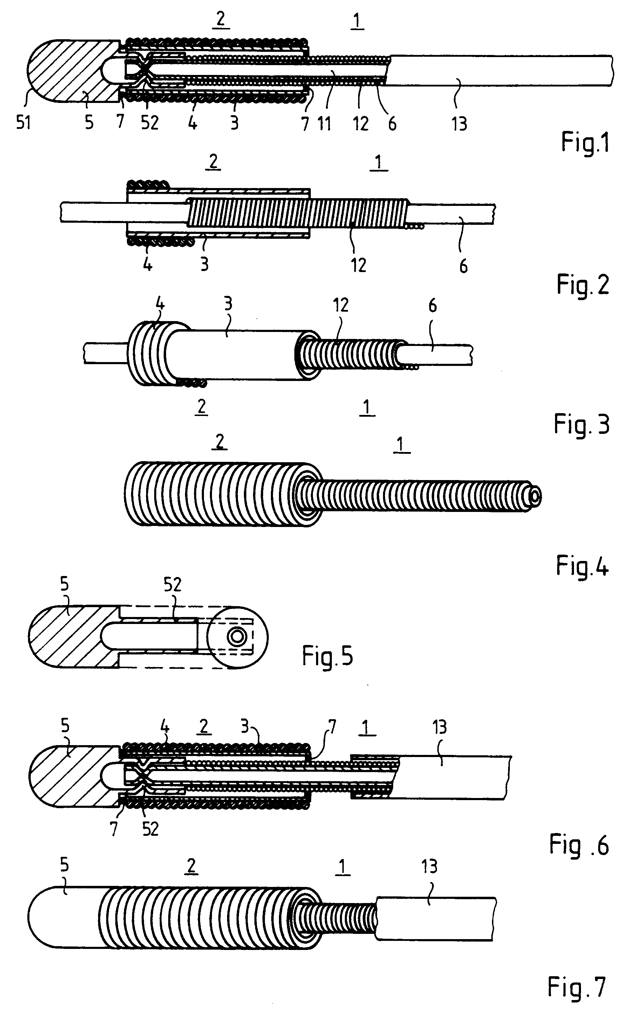

Bei dem in Figur 1 dargestellten Ausführungsbeispiel der erfindungsgemäßen Elektrode besteht der elektrisch leitende Bereich 2 der Elektrode aus einer zylinderförmigen Metallhülse 3 und einem um die Metallhülse 3 wendel- oder spiralförmig gewickelten Leiter 4, der bspw. aus demselben Material bestehen kann wie der elektrische Leiter 12 des Elektrodenleiters 1.

Der Elektrodenleiter 1 besteht aus mehreren dünnen Drahtwendeln 12, vorzugsweise einem vierwendeligen Leiter mit vier nebeneinander gewickelten Drahtwendeln. Die Drahtwendeln 12 schließen einen zentralen Hohlraum oder Lumen 11 ein, in den ein Mandrin zum Plazieren der Elektrode an einer vorbestimmten Stelle eingeführt werden kann.

Der leitfähige Bereich 2 der Elektrode wird an seinem äußeren Ende von einem Elektrodenkopf 5 abgeschlossen, der eine halbkugelförmige Spitze 51 und eine im Innern der zylinderförmigen Metallhülse 3 plazierte, zylindrische Klemmverbindung 52 aufweist. Die Klemmverbindung zwischen dem Elektrodenleiter 1 und dem Elektrodenkopf 5 wird durch einfaches Zusammenquetschen der Klemmverbindung 52 des Elektrodenkopfes 5 hergestellt.

Anhand der Figuren 2 bis 7 sollen die einzelnen Schritte zur Herstellung der erfindungsgemäßen Elektrode näher erläutert werden.

In den Figuren 2 und 3 ist im Schnitt bzw. in Draufsicht der Elektrodenleiter 1 und der leitfähige Bereich 2 der Elektrode dargestellt. Die zylindrische Metallhülse 3 ist in diesem Fertigungsstadium teilweise mit einem vierwendeligen Leiter 4 umwickelt, wobei die Wickelrichtung von der Spitze der Elektrode zur Einführöffnung für den Elektrodenleiter 1 verläuft. Der Elektrodenleiter 1 ist um einen Mitteldraht 6 gewickelt, der wahlweise im Elektrodenleiter 1 verbleiben kann oder nach dem Umwickeln zur Schaffung eines Lumen entfernt wird. Der Elektrodenleiter 1 weist ebenfalls einen vierwendligen Leiter 12 auf.

Nach dem vollständigen Umwickeln der zylindrischen Metallhülse 3 mit einem vierwendligen Leiter 4 wird die Elektrodenspitze 5 auf den Elektrodenleiter 1 aufgesteckt, der zu diesem Zweck durch die Metallhülse 3 durchgeschoben wird und die Vorderseite des leitfähigen Bereichs 2 um eine bestimmte Länge überragt. die ausreicht, um die Klemmverbindung 52 der Elektrodenspitze 5 aufzunehmen.

Der in Figur 5 dargestellte Querschnitt durch den Elektrodenkopf 5 zeigt eine halbkugelförmige Spitze 51 und eine hülsenförmig nach hinten fortgesetzte Klemmverbindung 52. Die auf den Elektrodenleiter 1 aufgesteckte Klemmverbindung 52 wird gemäß Figur 6 ringförmig zusammengepreßt und spannt den Elektrodenleiter 1 zwischen sich ein.

Anschließend wird ein Isolierschlauch 13 aus Silikonkautschuk, Polyurethan oder Polyäthylen vom rückwärtigen Ende des Elektrodenleiters 1 aufgeschoben (Figur 6, Figur 7).

Die Elektrodenspitze 5 besteht ebenfalls aus Metall, vorzugsweise demselben, körperverträglichen Material wie die wendelförmigen Leiter 4 bzw. die wendelförmigen Leiter 12 des Elektrodenleiters 1.

Zur Verbesserung der elektrischen Verbindung zwischen dem im Innern der zylindrischen Metallhülse 3 angeordneten Elektrodenleiter 1 und der Metallhülse 3 bzw. dem vierwendligen Leiter 4 am äußeren Umfang der Metallhülse 3 wird eine Schrauben-, Löt- oder Schweißverbindung 7 an der Stirnseite der Metallhülse 3 und der Rückseite des Elektrodenkopfes 5 vorgesehen. Zusätzlich oder alternativ kann eine Verbindung am rückseitigen Ende der Metallhülse 3, bspw. in Form einer Schweiß- oder Lötverbindung hergestellt werden.

Die Länge der zylindrischen Metallhülse 3 bzw. des leitfähigen Bereichs 2 der Elektrode beträgt ca. 25mm, bei einem Durchmesser von ca. 2 - 3, vorzugsweise 2,2mm.

Die vorbeschriebene Elektrode kann sowohl als bipolare als auch als monopolare Elektrode verwendet werden, wobei sie in diesem Falle als Anode dient und eine Kathode in an sich bekannter Weise verankert wird.

Bevorzugt eignet sich die erfindungsgemäße Elektrode für eine Plazierung im Bereich des Brustbeins sowohl in Verbindung mit einer indifferenten Elektrode bei unipolaren Herzschrittmachern als auch in Verbindung mit einer Referenzelektrode bei bipolaren Schrittmachern, wobei die Lage der Referenzelektrode auf eine Position innerhalb des Herzens beschränkt ist, während die indifferente Elektrode bei unipolaren Herzschrittmachern durch das Metallgehäuse des Schrittmachers gebildet wird.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Implantierbare Elektrode zur Herzstimulation mit einem isolierten Elektrodenleiter, der einerseits mit einem Herzschrittmacher verbindbar ist und andererseits einen mit einem Elektrodenkopf versehenen, elektrisch leitfähigen Bereich aufweist, der zylindrisch ausgebildet ist und einen wendelförmig gestreckten Leiter (4) aufweist, der koaxial über eine den Elektrodenleiter (1) bedeckende zylindrische Metallhülse (3) gewickelt ist, die auf den Elektrodenleiter (1) aufgesteckt und mit ihm elektrisch verbunden ist, wobei die zylindrische Metallhülse (3) an ihrem vorderen Ende mit dem in dieselbe eingesteckten Elektrodenkopf (5) versehen ist,
**dadurch gekennzeichnet,** daß
der Elektrodenkopf (5) mittels einer den Elektrodenleiter (1) einspannenden Klemmverbindung (52) mit dem Elektrodenleiter (1) elektrisch und mechanisch direkt verbunden ist.

2. Elektrode nach Anspruch 1, **dadurch gekennzeichnet,** daß die zylindrische Metallhülse (3) mit vier parallel gewickelten elektrischen Leitern (4) spiralförmig umwickelt ist.

3. Elektrode nach Anspruch 2, **dadurch gekennzeichnet,** daß der Elektrodenkopf (5) an seinem äußeren, aus der zylindrischen Metallhülse (3) herausragenden Ende (51) abgerundet ist.

4. Elektrode nach einem der vorangehenden Ansprüche, **dadurch gekennzeichnet,** daß der Elektrodenleiter (1) mindestens einen wendelförmigen Leiter (12) aufweist, der ein Lumen (11) zum Einführen eines Mandrin zur Plazierung der Elektrode umschließt.

5. Elektrode nach Anspruch 4, **dadurch gekennzeichnet,** daß der mindestens eine wendelförmige Leiter (12) des Elektrodenleiters (1) maximal bis zum Beginn der über den Elektrodenleiter (1) gesteckten zylindrischen Metallhülse (3) von einem Isolierschlauch (13) umgeben ist.

6. Elektrode nach einem der vorangehenden Ansprüche, **gekennzeichnet durch** eine Länge von 25mm und einen Durchmesser von 2 - 3mm.

## Claims

1. An implantable electrode for heart stimulation, comprising an insulated electrode conductor which at one side can be connected to a heart pacemaker and at the other side has an electrically conductive section of cylindrical structure which is provided with an electrode head and has a helically extended conductor (4), wherein the conductor (4) is wound coaxially over a cylindrical metal sleeve (3) covering the electrode conductor (1), the sleeve (3) being placed over the electrode conductor (1) and electrically connected thereto, and wherein the cylindrical metal sleeve (3) is provided at its front end with the electrode head (5) inserted in said sleeve, characterised in that the electrode head (5) is directly, electrically and mechanically connected to the electrode conductor (1) by means of a clamped joint (52) gripping the conductor (1).

2. An electrode according to claim 1, characterised in that the cylindrical metal sleeve (3) has four parallel-wound electrical conductors (4) wound spirally around it.

3. An electrode according to claim 2, characterised in that the electrode head (5) is rounded at its outer end (51) projecting from the cylindrical metal sleeve (3).

4. An electrode according to any of the preceding claims, characterised in that the electrode conductor (1) has at least one helical conductor (12) which surrounds a cavity (11) for the insertion of a mandrel for positioning the electrode.

5. An electrode according to claim 4, characterised in that the at least one helical conductor (12) of the electrode conductor (1) is surrounded by an insulating tube (13), at the maximum to the beginning of the cylindrical metal sleeve (3) placed over the electrode conductor (1).

6. An electrode according to any of the preceding claims, characterised by a length of 25 mm and a diameter of 2 - 3 mm.

## Revendications

1. Electrode implantable de stimulation cardiaque, comportant un conducteur d'électrode isolé connectable d'une part à un stimulateur cardiaque et présentant d'autre part une zone conductrice de l'électricité munie d'une tête d'électrode, de forme cylindrique et qui présente un conducteur (4) hélicoïdal enroulé de façon coaxiale sur une gaine métallique cylindrique (3) couvrant le conducteur d'électrode (1) et qui est enfilée sur le conducteur d'électrode (1) et lui est connectée électriquement, la gaine métallique cylindrique (3) étant dotée à son extrémité antérieure de la tête d'électrode (5) enfichée dans cette dernière, caractérisée en ce que la tête d'électrode (5) est connectée directement, électriquement et mécaniquement, au conducteur d'électrode (1) au moyen d'un raccord par serrage (52) enserrant le conducteur d'électrode (1).

2. Electrode selon la revendication 1, caractérisée en ce que la gaine métallique cylindrique (3) est guipée en spirale par quatre conducteurs électriques (4) enroulés parallèlement.

3. Electrode selon la revendication 2, caractérisée en ce que la tête d'électrode (5) est arrondie à son extrémité externe (51), dépassant de la gaine métallique cylindrique (3).

4. Electrode selon l'une des revendications précédentes, caractérisée en ce que le conducteur d'électrode (1) présente au moins un conducteur (12) hélicoïdal cernant une lumière (11) destinée à l'introduction d'un mandrin en vue de la mise en place de l'électrode.

5. Electrode selon la revendication 4, caractérisée en ce que ledit conducteur hélicoïdal (12) du conducteur d'électrode (1) est enveloppé au maximum jusqu'au début de la gaine métallique cylindrique (3) enfilée sur le conducteur d'électrode (1) par un tube souple isolant (13).

6. Electrode selon l'une des revendications précédentes, caractérisée par une longueur de 25 mm et un diamètre de 2 à 3 mm.
